# EUROPEAN PATENT SPECIFICATION

(11) **EP 2 512 436 B1**
(45) Date of publication and mention of the grant of the patent: **07.04.2021**
(21) Application number: 10795245.9
(22) Date of filing: 15.12.2010
(51) Int. Cl.: A61K 8/898, A61Q 5/02, A61Q 5/12, A61K 8/891, A61Q 5/00, A61Q 5/06

(54) **CONDITIONING COMPOSITION FOR HAIR**
ZUSAMMENSETZUNG ZUR HAARKONDITIONIERUNG
COMPOSITION DE SOIN CAPILLAIRE

(30) Priority: 18.12.2009 EP 09015677
(43) Date of publication of application: 24.10.2012
(73) Proprietor: Kao Germany GmbH, 64297 Darmstadt (DE)
(72) Inventor: MOLENDA, Michael, 60318 Frankfurt (DE); TIETJEN, Ilka, 68549 Ilvesheim (DE); GRIT, Mustafa, 64579 Gernsheim (DE)
(74) Representative: Grit, Mustafa
(86) International application number: PCT/EP2010/007679
(87) International publication number: WO 2011/072854

(56) References cited:
- EP-A1- 2 022 478
- WO-A1-2004/010967
- WO-A1-2004/032887
- WO-A1-2004/066972
- WO-A2-2011/009712
- JP-A- 1 203 314
- US-A1- 2009 255 063
- HERRWERT S ET AL: "Silicone Quaternium-22: New silicone technology for premium hair conditioning with additional benefits", SOFW-JOURNAL SEIFEN, OELE, FETTE, WACHSE, VERLAG FUR CHEMISCHE INDUSTRIE, AUGSBURG, DE, vol. 135, no. 6, 1 June 2009 (2009-06-01), pages 11-12,14, XP002584466, ISSN: 0942-7694
- GOTTSCHALCK T E ET AL: "INTERNATIONAL COSMETIC INGREDIENT DICTIONARY AND HANDBOOK, passage", 1 January 2006 (2006-01-01), INTERNATIONAL COSMETIC INGREDIENT DICTIONARY AND HANDBOOK, XX, XX, PAGE(S) 2148 - 2152, XP002470374, the whole document
- JURCZYK MATTHEW F ET AL: "CATIONIC SURFACTANTS AND QUATERNARY DERIVATIVES FOR HAIR AND SKIN CARE", 1 January 1999 (1999-01-01), COSMETIC SCIENCE AND TECHNOLOGY SERIES, DEKKER, NEW YORK, NY, US, PAGE(S) 223 - 249, XP001249398, ISSN: 0887-6541 pages 242-243
- DATABASE GNPD [Online] MINTEL; 1 May 2008 (2008-05-01), Kao Brands Jon Frieda: "Thickening Conditioner", Database accession no. 909702

## Description

The present invention is related to aqueous conditioning composition for hair comprising silicone quaternium-22 and at least one additional silicone compound which is different from the former one selected from arylated silicones. Conditioning composition of the present invention can be in the form of a shampoo, cleansing - conditioning composition, or in the form of a conditioner used after washing hair with cleansing compositions.

Conditioning compositions for hair have been known for ages. Various types of conditioners are available on the market and new ones are being introduced almost every day. Although this extremely developed conditioner market for hair, there is still need for improvements.

EP2022478 discloses conditioning compositions for hair comprising arylated silicone and at least one silicone quaternary compound such as silicone quaternium-18.

Silicone quaternium-22 was mentioned in "New Silicone Technology for Premium Hair Conditioning with Additional Benefits" (SOFW-Journal, 135, 6-2009).

Among important properties of hair, volume, body and shine are the ones very often addresses. Especially long lasting volume, body and shine are wished as they increase attractiveness of hair and change its perception. Volume and body giving products as well as shine enhancing ones are well known on the market either in a leave-in or rinse off applications.

Another important factor of effecting shiny appearance of hair and its volume and body is hair damage. As a rule damaged hair is less shiny and losses its volume and body easily than healthy hair because the damaging influences on hair destroy principally cuticle layer of hair. Therefore, it is important to use conditioning compositions in order to diminish and if this is not possible slow down hair damage.

On the other hand, conditioning compositions designed for damaged hair, especially damaged fine hair, often causes loss of volume and body although shiny appearance is observed to a certain extend. Compositions are beneficial which enhances hair shine and at the same time give hair, especially fine hair, volume and body.

In real life, people usually have hair with various damage levels in its length. Conditioner designed for damaged hair may, therefore, not be good enough or inappropriate for the healthy parts. Therefore, it is highly desirable to have conditioning compositions improving hair properties of damaged and healthy hair equally good so that consumer satisfaction is increased. In other words, it is highly beneficial for consumers to have a composition which conditions damaged and healthy hair homogeneously.

Therefore, first objective of the present invention is to provide aqueous conditioning compositions for hair which conditions damaged and healthy hair homogeneously. It has also been observed that by the use of the compositions of the present invention hair shine and volume and body are especially improved. Furthermore, other properties of hair are maintained or improved as well such as elasticity, manageability, smoothness and softness.

It has surprisingly been found out that aqueous composition comprising silicone quaternium-22 and at least one additional silicone compound which is different from the former one selected from arylated silicones conditions damaged and healthy hair homogeneously and gives hair shine, volume and body and hair treated with such a composition looks attractive and has its natural excellent shine, volume and body, elasticity, smoothness and it is easily manageable.

Accordingly the first object of the present invention is aqueous conditioning composition for hair comprising silicone quaternium-22 and at least one additional silicone compound which is different from the former one selected from arylated silicones.

Further object of the present invention is the use of the compositions of the present invention to condition hair comprising damaged and healthy parts in its length homogeneously, especially in terms of shine, volume and body, elasticity and manageability of hair.

It has also been observed during the course of tests that the effects are more pronounced when both cleansing and conditioning compositions and conditioning composition without any cleansing effect comprise silicone quaternium-22 and at least one additional silicone compound which is different from the former one selected from arylated silicones. Thus, further object of the present invention is a process for cleansing and conditioning hair wherein a cleansing and conditioning composition is applied onto hair and after rinsing off a conditioning composition without any cleansing effect is applied and optionally rinsed off from hair wherein both compositions comprise silicone quaternium-22 and at least one additional silicone compound which is different from the former one selected from arylated silicones.

Further, according to the above process, the object of the present invention is kit for cleansing and conditioning hair comprising a composition with at least one cleansing and foaming surfactant and silicone quaternium-22 and at least one additional silicone compound which is different from the former one selected from arylated silicones and a second composition comprising as well silicone quaternium-22 and at least one additional silicone compound which is different from the former one selected from arylated silicones.

Compositions of the present invention comprise silicone quaternium-22
which is commercially available under the trade name Abil T Quat 60 from Evonik Goldschmidt GmbH.

Composition of the present invention comprises silicone quaternium-22 at a concentration of 0.01 to 10%, preferably 0.02 to 7.5%, more preferably 0.05 to 5% and most preferably 0.1 to 3% by weight, calculated to total composition.

Compositions of the present invention comprise at least one additional silicone compound which is different from the above mentioned first silicone compound selected from arylated silicones.

Suitable arylated silicones are phenyl methicone, phenyl trimethicone, diphenyl dimethicone, diphenylsiloxy phenyl trimethicone, tetramethyl tetraphenyl trisiloxane, triphenyl trimethicone, and trimethyl pentaphenyl trisiloxane.

Concentration of at least one additional silicone selected from arylated siliones is in the range of 0.01 to 10%, preferably 0.02 to 7.5%, more preferably 0.05 to 5% and most preferably 0.1 to 3% by weight, calculated to total composition..

The compositions of the present invention can be either a conditioning -cleansing composition - shampoo - or a conditioning composition typically used after use of cleansing compositions.

Compositions of the present invention, the ones without cleansing effect, are suitable for either rinse off or leave in applications. Further object of the present invention is a process for conditioning hair wherein a composition according to present invention is applied onto hair and optionally rinsed off.

The composition of the present invention comprises additional hair-conditioning agents in any type of composition. Conditioning agents can be selected from oily substances, non-ionic substances, cationic amphiphilic ingredients, cationic polymers or their mixtures.

Oily substances are selected from such as natural oils such as olive oil, almond oil, avocado oil, wheatgerm oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Concentration of one or more oily substances is in the range of 0.01 to 10%, preferably 0.05 to 7.5%, more preferably 0.1 to 5 and most preferably 0.1 to 3% by weight calculated to total composition. The concentrations referred here are total concentration of all oily substances may be present in the composition.

Non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₃ CO (O CH₂ CH₂)ₙ OH

or

R₃ CO (O CH₂ CH₂)ₙ O OC R₄

where R₃ and R₄ are independent from each other saturated, unsaturated or branched or non-branched alkyl chain with 7 to 21 C atoms and n is typically 2 - 100.

Concentration of one or more non-ionic conditioning agents is in the range of 0.01 to 10%, preferably 0.05 to 7.5%, more preferably 0.1 to 5 and most preferably 0.1 to 3% by weight calculated to total composition. The concentrations referred here are total concentration of all non-ionic conditioning agents may be present in the composition.

In one of the preferred from of the present invention, conditioning compositions comprise at least one cationic polymer as conditioning agent. Suitable cationic polymers are those of best known with their CTFA category name Polyquaternium. Typical examples of those are Polyquaternium 1, Polyquaternium 2, Polyquaternium 4, Polyquaternium 5, Polyquaternium 6, Polyquaternium 7, Polyquaternium 8, Polyquaternium 9, Polyquaternium 10, Polyquaternium 11, Polyquaternium 12, Polyquaternium 13, Polyquaternium 14, Polyquaternium 15, Polyquaternium 16, Polyquaternium 17, Polyquaternium 18, Polyquaternium 19, Polyquaternium 20, Polyquaternium 22, Polyquaternium 24, Polyquaternium 27, Polyquaternium 28, Polyquaternium 29, Polyquaternium 30, Polyquaternium 31, Polyquaternium 32, Polyquaternium 33, Polyquaternium 34, Polyquaternium 35 and Polyquaternium 36, Polyquaternium-37, Polyquaternium 39, Polyquaternium 42, Polyquaternium 43, Polyquaternium 44, Polyquaternium 45, Polyquaternium 46, Polyquaternium 47, Polyquaternium 48, Polyquaternium-49, Polyquaternium 50, Polyquaternium 51, Polyquaternium 52, Polyquaternium 53, Polyquaternium 54, Polyquaternium 55, Polyquaternium 56, Polyquaternium 57, Polyquaternium 58, Polyquaternium 59, Polyquaternium 60, Polyquaternium 61, Polyquaternium 62, Polyquaternium 63, Polyquaternium 64, Polyquaternium 65, Polyquaternium 66, Polyquaternium 67, Polyquaternium 68, Polyquaternium 69, Polyquaternium-70, Polyquaternium 71, Polyquaternium 72, Polyquaternium 73, Polyquaternium 74, Polyquaternium 75, Polyquaternium 76, Polyquaternium 77, Polyquaternium 78, Polyquaternium-79, Polyquaternium 80, Polyquaternium 81, Polyquaternium 82, Polyquaternium 83, Polyquaternium 84, Polyquaternium 85, Polyquaternium 86 and Polyquaternium 87.

It has further been found out that especially those of cationic cellulose type polymers known as Polymer JR type from Amerchol such as Polyquaternium 10 or cationic galactomannans such as cationic guar gum known with trade name Jaguar from Rhone-Poulenc which are chemically for example Guar hydroxypropyl trimonium chloride and cationic tara gum an its derivatives known with INCI name Caesalpinia spinosa hydroxypropyltrimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The most preferred cationic polymers are those of cationic cellulose derivatives, cationic guar gum derivatives, cationic Caesalpinia spinosa gum derivatives, polyquaternium 6, polyquaternium 7, polyquaternium 67 and polyquaternium 70.

Conditioning compositions of the present invention can comprise additionally one or more cationic surfactant(s) as conditioner presented with the general formula 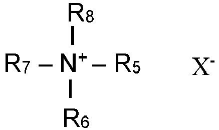 where R₅ is a saturated or unsaturated, branched or non-branched alkyl chain with 8-24 C atoms or

R₉ CO NH (CH₂)ₙ

where R₉ is saturated or unsaturated, branched or non-branched alkyl chain with 7-21 C atoms and n has value of 1 - 4,
or

R₁₀ CO O (CH₂)ₙ

where R₁₀ is saturated or unsaturated, branched or non-branched alkyl chain with 7-21 C atoms and n has value of 1 - 4, and
R₆ is hydrogen or unsaturated or saturated, branched or non-branched alkyl chain with 1 - 24 C atoms or

   R₉ CO NH (CH₂)ₙ

   or

   R₁₀ CO O (CH₂)ₙ

   where R₉, R₁₀ and n are same as above.
R₇ and R₈ are hydrogen or lower alkyl chain with 1 to 4 carbon atoms which may be substituted with one or more hydroxyl group, and X is anion such as chloride, bromide, methosulfate.

Typical examples of those ingredients are cetyltrimethyl ammonium chloride, steartrimonium chloride, behentrimonium chloride, stearamidopropyl trimonuim chloride, dioleoylethyl dimethyl ammonium methosulfate, dioleoylethyl hydroxyethylmonium methosulfate.

Amido amines may as well be used as a conditioning cationic surfactant in the compositions of the present invention. Typical non-limiting example is stearamidopropyl dimethyl amine known with a trade name Tego Amid S18 from Degussa and Lexamine S13 from Inolex.

The compositions according to the invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

Typical concentration range for any of those conditioners of cationic polymers, and cationic surfactants is in the range of 0.01 - 10% by weight, preferably 0.01 - 7.5% by weight, more preferably 0.05 - 5% and most preferably 0.1 - 3% by weight calculated to the total composition. It should be noted that especially non-cleansing conditioning type of the products contain higher concentrations of the above mentioned concentrations of the cationic surfactants which at the same time if desired can be emulsifying agent. In cleansing and conditioning type of preparations, concentration of cationic surfactants is lower.

Conditioning composition of the present invention comprises at least one glyceryl ether of the following formula 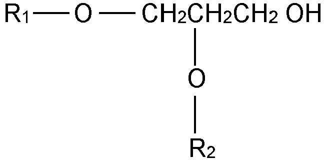 wherein R₁ is straight or branched, saturated or unsaturated alkyl chain with 4 to 24 C atoms, preferably 4 to 18 and more preferably 4 to 12 C atoms and R₂ is H, or straight or branched, saturated or unsaturated alkyl chain with 4 to 24 C atoms, 4 to 18 and more preferably 4 to 12 C atoms and most preferably R₅ is H, at a concentration of 0.1 to 10%, preferably 0.1 to 5% and more preferably 0.25 to 3% and most preferably 0.5 to 2.5% by weight calculated to total composition.

Suitable unlimited examples are glyceryl butyl ether, glyceryl isobutyl ether, glyceryl tert-butyl ether, glyceryl pentyl ether, glyceryl isopentyl ether, glyceryl hexyl ether, glyceryl isohexyl ether, glyceryl heptyl ether, glyceryl octyl ether, glyceryl ethylhexyl ether, glyceryl nonyl ether, glyceryl decyl ether, glyceryl isodecyl ether, glyceryl lauryl ether, glyceryl myristyl ether, glyceryl palmityl ether, glyceryl stearyl ether and glyceryl behenyl ether and their mixtures. Most preferred are glyceryl butyl ether, glyceryl isobutyl ether, glyceryl tert-butyl ether, glyceryl pentyl ether, glyceryl isopentyl ether, glyceryl hexyl ether, glyceryl isohexyl ether, glyceryl heptyl ether, glyceryl octyl ether, glyceryl ethylhexyl ether, glyceryl nonyl ether, glyceryl decyl ether, glyceryl isodecyl ether are glyceryl lauryl ether, and their mixtures.

It should be noted that within the disclosure of the present description, gylceryl decyl ether is used as synonym of decyl glycerine. For the other compounds in the above paragraph the same is valid.

Composition of the present invention can preferably comprise at least one polyphenol. With the word polyphenol it is meant that an organic molecule with at least 2 hydroxyl groups in its molecule.

In the preferred from of the invention, at least one polyphenol or mixture of polyhenols is included into compositions of the present invention from a natural plant extract. In principal any natural plant extract rich of polyphenols is suitable within the meaning of the present invention. Within the meaning of the present invention the extracts are liquid extracts and prepared by mixing plant parts such as leaves, fruits, blossoms and roots with a solvent such as water, alcohol, propyleneglycol or mixture of more than one solvent and incubating for certain period of time and filtrating the undissolved plant parts. Suitable aqueous (e.g. steam-distilled) alcoholic or hydro-alcoholic plant extracts known **per se** are in particular aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc. Suitable trade products are, for example, the various "Extrapon®" products, "Herbasol^{R}", "Sedaplant^{R}" and "Hexaplant^{R}". Extracts and the preparation thereof are also described in "Hagers Handbuch der pharmazeutischen Praxis", 4th Ed. Preferred plant extracts are prepared from Vitis vinifera, Malus domestica, Camelia sinensis, Juglans regia Ribes Uva-Crispa, Ribes nigrum, Ribes rubrum and Punica granatum. The above mentioned extracts may also be available in the powder form and such are also suitable within the meaning of the present invention.

The polyphenol comprising extracts are included into the compositions of the present invention at a concentration of 0.001 to 10%, preferably0.005 to 7.5%, more preferably 0.01 to 5% and most preferably 0.05 to 2.5% by weight, calculated to total composition based on dry matter of the extract.

In another preferred form of the invention, conditioning composition comprises one or more organic solvent such as ethanol, propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylenecarbonate, propyleneglycol, poypropyleneglycols, ethyleneglycol monoethylether, ethylene glycol monobutyl ether, ethylene glycol monophenyl ether, 1-phenylethylalcohol, 2-phenylethylalcohol, o-methoxyphenol. The most preferred ones are benzylalcohol and polypropylene glycols. Concentration of organic solvents should not exceed 10% by weight, preferably in the range of 0.1 to 7.5%, more preferably 0.1 to 5% by weight and most preferably 0.1 to 3% by weight calculated to total composition.

Further in preferred embodiment of the present invention, compositions comprise at least one UV filter and at least one ubichinone of the following formula 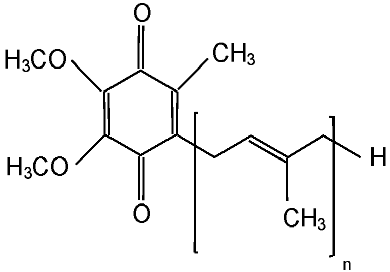 where n is a number between 1 and 10. It should be noted that the compositions of the present invention can certainly comprise more than one ubichinone.

Preferred ubichinones are the ones where n is a number between 6 and 10 and especially preferred is Ubichinone 50 where n is 10, also known as Coenzyme Q10. Concentration ubichinone of the above formula in the compositions is from 0.0001 to 1%, preferably from 0.0002 to 0.75%, more preferably from 0.0002 to 0.5% and most preferably from 0.0005 to 0.5% by weight, calculated to total composition.

Compositions of the present invention preferably comprise at least one UV filter. Principally any substance known as UV filter is suitable for the compositions of the present invention. Non-limiting examples are 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxy- benzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof, 3-(4'-methyl benzylidene)-DL-campher, and/or polysilicone-15. Above mentioned UV filters are those oil and water soluble ones for the purpose of protecting hair colour. In other words, anionic and nonionic, oily, UV filters are suitably used in the compositions of the present invention. In the preferred from of the invention the compositions comprise at least one water soluble UV filter and at least one oil soluble one. Further preferred that both UV filters are present at a weight ratio in the range of oil soluble to water soluble UV filter 1:10 to 10:1, preferably 1:5 to 5:1, more preferably 1:3 to 3:1 and most preferably 1:1 in the compositions of the present invention.

The amount of the UV-absorber as a total ranges typically from about 0.01 % to 5%, preferably 0.05 to 3%, more preferably from 0.05 % to 2.5% and most preferably from 0.1 % to 2 % by weight, calculated to the total composition. Further in preferred embodiment of the present invention, compositions comprise at least one direct dye. Suitable direct dyes are of cationic, anionic and neutral nitro dyes. It should be noted that they can also be used in combination with each other. In other words a composition according to present invention can comprise an anionic and a cationic dye as well as an anionic and a nitro dye or a cationic and a nitro dye. Certainly the combination of all three dyestuffs is also possible. Any cationic direct dye is in principal suitable for the compositions. Examples are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Orange 31, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87.

Any anionic dye is in principal suitable for the compositions. Suitable examples are such as Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Among those, the preferred anionic dyestuffs are Acid Red 52, Acid Violet 2, Acid Red 33, Acid Orange 4, Acid Red 27 and Acid Yellow 10 and their salts. The most preferred anionic dyes are Acid Red 52, Acid Violet 2, Acid Red 33, Acid Orange 4 and Acid Yellow 10, and their

Neutral dyes, so called nitro dyes for shading purposes are also optionally contained in the compositions. Suitable ones are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Concentration of one or more direct dyes in total is in the range of 0.001 to 5% by weight, preferably 0.01 to 4% more preferably 0.05 to 3% and most preferably 0.1 to 2.5% by weight calculated to total composition.

Compositions of the present invention comprise additionally at least one fatty alcohol of the following formula

R₁₅-OH

where R₁₅ is a saturated or unsaturated, branched or non-branched fatty acyl chain with 8 - 24 C atoms. Concentration of fatty alcohols is usually less than 20%, preferably less than 15% by weight calculated to total composition. Typical examples to the most useful fatty alcohols are myristyl alcohol, palmityl alcohol, cetyl alcohol, stearyl alcohol, behenyl alcohol and their mixtures. As a mixed fatty alcohol the mostly used one is the cetearyl alcohol as well preferred in the compositions of the present invention.

Conditioning compositions of the present invention can be a cleansing composition (cleansing-conditioning composition). Cleansing conditioning compositions of the present invention comprise at least one surfactant selected from anionic, non-ionic and/or amphoteric or zwitterionic surfactants at a concentration range of 5 to 50%, preferably 5 to 40% and more preferably 5 to 30%, and most preferably 5 to 25% by weight, calculated to the total composition.

In an embodiment of the present invention cleansing conditioning composition of the present invention, comprises at least one anionic, at least one nonionic surfactant. More preferably the compositions further comprise additionally at least one amphoteric surfactant.

Anionic surfactants suitable within the scope of the invention are preferably present in an amount from 1 to about 30%, preferably 2 to 20% and most preferably 2 - 15%, by weight, calculated to the total composition.

These are anionic surfactants of the sulfate, sulfonate, carboxylate and alkyl phosphate type, especially, of course, those customarily used in shampoo compositions, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates constituting mild, skin-compatible detergents.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₁₁-(C₂H₄O)ₙ-O-CH₂COOX,

wherein R₁₁ is a C₈-C₂₈-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 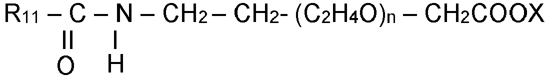 wherein R₁₁ and X have the above meanings, and n is in particular a number from 1 to 10, preferably 2.5 to 5.

Such products have been known for some time and are on the market, for example, under the trade name "AKYPO®" and "AKYPO-SOFT®".

Also useful are C₈-C₂₈-acyl isethionates, alone or in admixture with other anionic surfactants, as well as sulfofatty acids and the esters thereof.

It is also possible to use mixtures of several anionic surfactants, for example an ether sulfate and a polyether carboxylic acid or alkyl amidoether carboxylic acid.

An overview of the anionic surfactants used in liquid body cleansing compositions can furthermore be found in the monography of K. Schrader and A. Domsch, "osmetology - Theory and Practice", 2005, Verlag für chemische Industrie, Augsburg - Germany, pp. II-8-II-19.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants. Further surfactants in the conditioning - cleansing compositions according to the invention are nonionic surfactants, preferably in admixture with anionic surfactants.

These are described in Schrader, I.c., on pages 600-601 and pp. 694-695. Especially suited are alkyl polyglucosides of the general formula

R₁₂-O-(R₁₃O)ₙ-Zₓ,

wherein R₁₂ is an alkyl group with 8 to 18 carbon atoms, R₁₃ is an ethylene or propylene group, Z is a saccharide group with 5 to 6 carbon atoms, n is a number from 0 to 10 and x is a number between 1 and 5.

These alkyl polyglucosides have recently become known in particular as excellent skin-compatible, foam improving agents in liquid detergents and body cleansing compositions, and are present in an amount from about 1 % to 15 %, in particular from 1 % to 10 % by weight, calculated to the total composition.

Mixtures of anionic surfactants and alkyl polyglucosides as well as the use thereof in liquid body cleansing compositions are already known, for example, from EP-A 70 074. The alkyl polyglucosides disclosed therein are basically also suited within the scope of the present invention; as well as the mixtures of sulfosuccinates and alkyl polyglucosides disclosed in EP-A 358 216.

Further nonionic surfactant components are, for example, long-chain fatty acid mono- and dialkanolamides, such as coco fatty acid monoethanolamide and myristic fatty acid monoethanolamide, which can also be used as foam enhancers, preferably in amounts from about 1 % to about 5 % by weight.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates.

Further suitable nonionic surfactants are amineoxides which may be present in an amount from 0.25 % to 5 % by weight, calculated to the total composition.

Such amineoxides are state of the art, for example C₁₂-C₁₈- alkyl dimethyl amineoxides such as lauryl dimethyl amineoxide, C₁₂-C₁₈- alkyl amidopropyl or - ethyl amineoxides, C₁₂-C₁₈ -alkyl di(hydroxyethyl) or (hydroxypropyl) amineoxides, or also amineoxides with ethyleneoxide and/or propyleneoxide groups in the alkyl chain. Such amineoxides are on the market, for example, under the trade names "Ammonyx®", "Aromox®" or "Genaminox®".

Further nonionic surfactants useful in the cleansing conditioning compositions according to invention are C₁₀-C₂₂-fatty alcohol ethoxylates at a concentration of 0.5 to 10%, preferably 0.5 to 5% by weight, calculated to total composition. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":
The average degree of ethoxylation thereby ranges between about 2.5 and about 25, preferably about 10 and about 20.

As further surfactant component, the cleansing conditioning compositions according to the invention can also contain amphoteric or zwitterionic surfactants, for example in an amount from about 0.5 % to about 15 %, preferably from about 1 % to about 10 %, by weight, calculated to the total composition. It has especially been found out that addition of zwitterionic or amphoteric surfactants enhances foam feeling in terms of creaminess, foam volume and as well as skin compatibility is improved. For achieving milder formulations anionic surfactant, especially of sulphate types, to amphoteric surfactant ratio should be in the range of 10:1 to 1:1, preferably 5:1 to 1:1.

Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

In detail, it is possible to use betaines of the structure 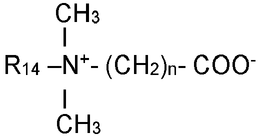 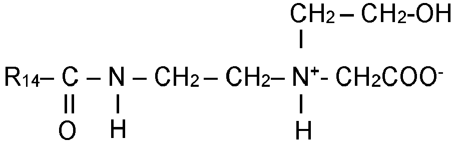 wherein R₁₄ is a C₈-C₁₈-alkyl group and n is 1 to 3;
sulfobetaines of the structure 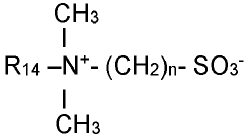 wherein R₁₄ and n are same as above;
and amidoalkyl betaines of the structure 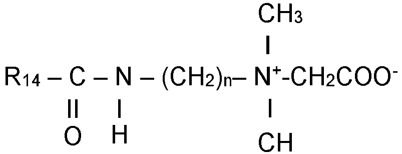 wherein R₁₄ and n are same as above.

Solubilizers may be added to the compositions, in particular cleansing compositions, especially when oily substances are chosen as conditioning agents and fragrance oils with highly lipophilic properties. Typical solubilizers may be hydrogenated castor oil known with the trade mark Cremophor RO series from BASF. It should be noted that as well the surfactant mixture can be a good solubilizer for fragrance oils. Typical concentration of the solubilizers can be in the range of 0.01 - 2% by weight, preferably 0.1 - 1% by weight, calculated to total composition.

Conditioning and cleansing composition of the present invention can be transparent as well as pearly. Transparency of the composition is judged by naked eye in a transparent shampoo bottle with a thickness not more than 5 cm. In the case a transparent appearance is wished, the following ingredients are not essential. However, pearl-shiny appearance is achieved with those dispersed in cleansing color-enhancing compositions in crystalline form, i.e. so called pearl-shine or pearlizing agents. The preferred once are PEG-3 distearate and ethylene glycol distearate. The concentration of those can typically be from 0.1 to 3%, preferably 0.5 to 2% by weight, calculated to the total composition. These compounds are preferably added to the compositions in admixture with anionic, nonionic and/or amphoteric surfactants. Such kind of mixtures is available commercially.

Hair cleansing conditioning compositions of the present invention can be in the form of conventional liquid thickened shampoo, as well in the form of ready to use foam, delivered either from a pump-foamer or from an aerosol bottle. In the case that an aerosol foam preparation is preferred, propellant gas must be added to the formulation. The suitable propellant gasses are carbondioxide, dimethylether and alkanes such as butane propane or their mixtures.

Conditioning compositions of the present invention can be in the form of emulsions, solutions, gels and dispersions. In the case that solutions and/or gels forms are preferred the appearance can be either with a transparent or opaque.

As a product form, foam is as well suited when packed into a pressurized can or delivered through a pump-foamer (non-aerosol). In the case that an aerosol foam preparation is preferred, propellant gas must be added to the formulation. The suitable propellant gasses are carbondioxide, dimethylether and alkanes such as butane, propane, isobutane or their mixtures.

Conditioning compositions of the present invention can comprise moisturizers, chelating agents, preservatives and fragrance. The moisturizing agents are selected from panthenol, polyols, such as glycerol, polyethylene glycols with molecular weight 200 to 20,000. The moisturizing ingredients can be included in the conditioner compositions at a concentration range of 0.01 - 2.5% by weight calculated to the total composition.

The sequestering agents are selected from polycarboxy acids. The preferred one is ethylene diamine tetraacetic acid, EDTA. Typical useful concentration range for sequestering agents is of 0.01 - 2.5% by weight calculated to the total composition.

The pH of the compositions according to the present invention is suitably between 2 and 8 and preferably in the range of 2.5 to 6.5, more preferably 3 to 5.5 and most preferably 3.5 to 5.

In principal pH of the compositions can be adjusted with any organic and/or inorganic acids or their mixture. Some of them to mention are phosphoric acid, hydrochloric acid as the inorganic ones and to the organic acids the well known citric acid and lactic acid, glycolic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid. It has further been observed that improved conditioning and bnrightening performace was observed when compositions comprise at the same time at least one hydroxycarboxylic and/or dicarboxylic acids.

The viscosity of the conditioning shampoo compositions according to the invention is in the range of 500 and about 20,000 mPa.s at 20°C, preferably 1,000 to 10,000, in particular 1,500 to 8,000 mPa.s at 20°C, measured with Höppler viscosimeter.

Viscosity of shampoo compositions can be adjusted with known viscosity enhancers. The preferred ones are glyceryl laurate, PEG-55 propyleneglycol oleate and PEG-18 glyceryl oleate/cocoate known with the trade names Antil^{R} 141 and 171, respectively and PEG-160 sorbitan triisostearate known with a trade name Rheodol^{R}. It should be noted that in the case that a composition are delivered in the form of a foam from a pump-foamer and/or aerosol can, those compositions should not be thickened and have a viscosity value not more than 500 mPa.s, more preferably 250 mPa.s measured as mentioned above at room temperature.

Viscosity of the non-cleansing conditioning composition should not be more than 50,000 mPa.s at 20°C measured with Brookfield Rheometer at a shear rate of 10 sec-1 .

The following examples are to illustrate the invention, but not to limit. The compositions according to the invention are prepared by mixing the individual components in water, whereby it is also possible to use pre-mixtures of various ingredients.

### Example 1

| | |
|---|---|
| Sodium lauryl ether sulfate | 11.0 (% by wt.) |
| Coco glucoside | 5.0 |
| Cocoamidopropyl betaine | 3.0 |
| Ethylhexyl glycerin | 0.7 |
| Phenyl trimethicone | 0.2 |
| Laureth-16 | 4.0 |
| Cationic polymer (Polyquaternium-11) | 0.5 |
| Benzophenone-3 | 0.2 |
| Benzylalcohol | 0.5 |
| Silicone quaternium - 22 | 0.4 |
| Ubichinone | 0.05 |
| Vitis vinifera (dry matter) | 0.20 |
| Lactic acid | q.s. to pH 5.0 |
| Perfume, preservative | q.s. |
| Water | q.s. to 100 |

The above composition is a very low viscosity composition, in any case a viscosity lower than 500 mPa.s measured at ambient temperature and with Höppler viscosimeter, confectioned into a pump-foamer as purchased from the company Air-Spray - Germany and showed excellent brightening and shine effect.

Similarly and aerosol foam shampoo was prepared by confectioning the above composition at a weight ratio of 90/10 - composition/propellant- using propane-butane mixture as a propellant. The foam shampoos so obtained showed excellent cleansing and brightening and shine effects.

Additionally, into the above shampoo 0.05% basic blue 99, and 0.005% basic red 51 was added. Excellent warm silver shine was observed on the washed gray hair. At the same time, excellent anti-yellow effect is observed on the freshly bleached hair.

Furthermore the above cleansing and conditioning composition was added 1% by weight sodium chloride and it became a thickened shampoo without loss of any effects mentioned above.

## Claims

1. Aqueous conditioning composition for hair **characterized in that** it comprises silicone quaternium-22 and at least one additional silicone compound which is different from the former one selected from arylated silicones.

2. Composition according to claim 1 **characterized in that** it comprises silicone quaternium-22 at a concentration of 0.01 to 10% by weight and at least one additional silicone compound which is different from the former one selected from arylated silicones at a concentration of 0.01 to 10% by weight, all values are calculated to total composition.

3. Composition according to claims 1 and/or 2 **characterized in that** it is an emulsion and comprises at least one fatty alcohol according to general formula
R₁₅-OH
where R₁₅ is a saturated or unsaturated, branched or non-branched fatty acyl chain with 8 - 24 C atoms.

4. Composition according to any of the preceding claims **characterized in that** it comprises cationic surfactant and/or cationic polymer.

5. Composition according to any of the preceding claims **characterized in that** it comprises at least one compound according to the formula 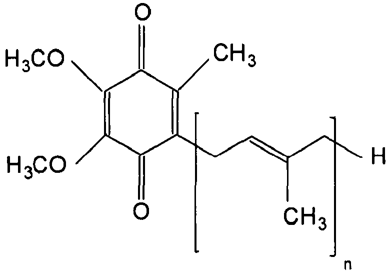 where n is a number between 1 and 10, and/or at least one UV filter and/or at least one polyphenol and/or at least one organic solvent.

6. Composition according to any of the preceding claims it comprises at least one direct dye.

7. Composition according to any of the preceding claims **characterized in that** compositions are cleansing and conditioning composition (shampoo) and comprising at least one surfactant selected from anionic, nonionic and amphoteric or zwitterionic surfactants at a concentration of 5 to 50% by weight calculated to the total composition, preferably it comprises at least one anionic surfactant and at least one non-ionic surfactant and at least one amphoteric surfactant.

8. Composition according to any of the preceding claims **characterized in that** it has a pH in the range of 2.0 to 8.0.

9. Use of the composition according to claims 1 to 8 for conditioning hair comprising damaged parts.

10. Kit for conditioning hair **characterized in that** it comprises a composition for cleansing hair comprising silicone quaternium - 22 and at least one additional silicone compound which is different from the former one selected from arylated silicones and a second composition without cleansing effect comprising silicone quaternium - 22 and at least one additional silicone compound which is different from the former one selected from arylated silicones.

## Patentansprüche

1. Wässrige Konditionierungszusammensetzung für Haare, **dadurch gekennzeichnet, dass** sie Silicone Quaternium-22 und mindestens eine weitere Silikonverbindung aufweist, welche eine andere als die erstere ist, ausgewählt aus arylierten Silikonen.

2. Zusammensetzung nach Anspruch 1, **dadurch gekennzeichnet, dass** sie Silicone Quaternium-22 in einer Konzentration von 0,01 Gewichts-% bis 10 Gewichts-% und mindestens eine weitere Silikonverbindung, welche eine andere als die erstere ist, ausgewählt aus arylierten Silikonen, in einer Konzentration von 0,01 Gewichts-% bis 10 Gewichts-% aufweist, wobei alle Werte auf die Gesamtzusammensetzung bezogen sind.

3. Zusammensetzung nach Anspruch 1 und/oder 2, **dadurch gekennzeichnet, dass** sie eine Emulsion ist und mindestens einen Fettalkohol gemäß der allgemeinen Formel
R₁₅-OH
aufweist, wobei R₁₅ eine gesättigte oder ungesättigte, verzweigte oder unverzweigte Fettalkylkette mit 8 bis 24 C-Atomen ist.

4. Zusammensetzung nach einem der vorhergehenden Ansprüche, **dadurch gekennzeichnet, dass** sie kationisches Tensid und/oder kationisches Polymer aufweist.

5. Zusammensetzung nach einem der vorhergehenden Ansprüche, **dadurch gekennzeichnet, dass** sie mindestens eine Verbindung gemäß der Formel 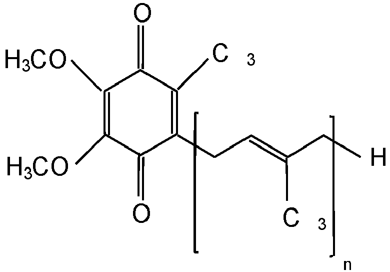 wobei n eine Zahl von 1 bis 10 ist, und/oder mindestens einen UV-Filter und/oder mindestens ein Polyphenol und/oder mindestens ein organisches Lösungsmittel aufweist.

6. Zusammensetzung nach einem der vorhergehenden Ansprüche, sie weist mindestens einen Direktfarbstoff auf.

7. Zusammensetzung nach einem der vorhergehenden Ansprüche, **dadurch gekennzeichnet, dass** Zusammensetzungen Reinigungs- und Konditionierungszusammensetzung (Shampoo) sind und mindestens ein Tensid, ausgewählt aus anionischen, nichtionischen und amphoteren oder zwitterionischen Tensiden, in einer Konzentration von 5 Gewichts-% bis 50 Gewichts-% aufweisen, bezogen auf die Gesamtzusammensetzung, vorzugsweise weist sie mindestens ein anionisches Tensid und mindestens ein nichtionisches Tensid und mindestens ein amphoteres Tensid auf.

8. Zusammensetzung nach einem der vorhergehenden Ansprüche, **dadurch gekennzeichnet, dass** sie einen pH-Wert im Bereich von 2,0 bis 8,0 aufweist.

9. Verwendung der Zusammensetzung nach Anspruch 1 bis 8 zum Konditionieren von Haaren, welche beschädigte Teile aufweisen.

10. Kit zum Konditionieren von Haaren, **dadurch gekennzeichnet, dass** es eine Zusammensetzung zum Reinigen von Haaren, die Silicone Quaternium-22 und mindestens eine weitere Silikonverbindung aufweist, welche eine andere als die erstere ist, ausgewählt aus arylierten Silikonen, und eine zweite Zusammensetzung ohne Reinigungswirkung aufweist, die Silicone Quaternium-22 und mindestens eine weitere Silikonverbindung aufweist, welche eine andere als die erstere ist, ausgewählt aus arylierten Silikonen.

## Revendications

1. Composition de soin aqueuse pour cheveux **caractérisée en ce qu'**elle comprend du silicone quaternium-22 et au moins un composé silicone supplémentaire qui est différent du précédent, choisi parmi des silicones arylés.

2. Composition selon la revendication 1, **caractérisée en ce qu'**elle comprend du silicone quaternium-22 à une concentration de 0,01 à 10 % en poids et au moins un composant silicone supplémentaire, qui est différent du précédent, choisi parmi des silicones arylés à une concentration de 0,01 à 10 % en poids, toutes les valeurs sont calculées par rapport à la composition totale.

3. Composition selon les revendications 1 et/ou 2, **caractérisée en ce qu'**elle est une émulsion et comprend au moins un alcool gras selon la formule générale
R₁₅-OH
où R₁₅ est une chaîne acyle grasse, saturée ou insaturée, ramifiée ou non ramifiée, avec 8 à 24 atomes C.

4. Composition selon l'une quelconque des revendications précédentes, **caractérisée en ce qu'**elle comprend un tensioactif cationique et/ou un polymère cationique.

5. Composition selon l'une quelconque des revendications précédentes, **caractérisée en ce qu'**elle comprend au moins un composé selon la formule 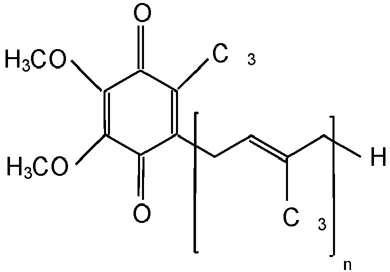 où n est un nombre entre 1 et 10, et/ou au moins un filtre UV, et/ou au moins un polyphénol, et/ou au moins un solvant organique.

6. Composition selon l'une quelconque des revendications précédentes, elle comprend au moins un colorant direct.

7. Composition selon l'une quelconque des revendications précédentes, **caractérisée en ce que** des compositions sont une composition de nettoyage et de soin (shampoing) et comprenant au moins un tensioactif choisi parmi des tensioactifs anioniques, non ioniques et amphotères ou zwitterioniques à une concentration de 5 à 50 % en poids, calculée par rapport à la composition totale, elle comprend de préférence au moins un tensioactif anionique et au moins un tensioactif non ionique, et au moins un tensioactif amphotère.

8. Composition selon l'une quelconque des revendications précédentes, **caractérisée en ce qu'**elle a un pH dans la plage de 2,0 à 8,0.

9. Utilisation de la composition selon les revendications 1 à 8 pour le soin des cheveux comprenant des parties endommagées.

10. Kit de soin pour cheveux **caractérisé en ce qu'**il comprend une composition pour nettoyer les cheveux comprenant du silicone quaternium-22 et au moins un composant silicone supplémentaire, qui est différent du précédent, choisi parmi des silicones arylés, et une seconde composition sans effet nettoyant comprenant du silicone quaternium-22 et au moins un composant silicone supplémentaire, qui est différent du précédent, choisi parmi des silicones arylés.
